(19) Europäisches Patentamt
European Patent Office
Office européen des brevets

(11) Veröffentlichungsnummer: **0 002 461**
**B1**

(12) # EUROPÄISCHE PATENTSCHRIFT

(21) Anmeldenummer: **78101458.4**

(22) Anmeldetag: **25.11.78**

(51) Int. Cl.³: **C 07 D 231/06, D 06 L 3/12**

(54) **Pyrazolin-Weisstöner, Verfahren zu ihrer Herstellung sowie ihre Verwendung zum Behandeln von Polyacrylnitril und Wolle**

(30) Priorität: **09.12.77 DE 2755023**

(43) Veröffentlichungstag der Anmeldung: **27.06.79 Patentblatt 79/13**

(45) Bekanntmachung des Hinweises auf die Patenterteilung: **23.07.80 Patentblatt 80/15**

(84) Benannte Vertragsstaaten: **CH DE FR GB**

(56) Entgegenhaltungen:
**DE - A - 2 011 552**
**FR - 6099 M**
**FR - A - 1 296 406**

(73) Patentinhaber: **Bayer AG**
**Zentralbereich Patente, Marken und Lizenzen**
**D - 5090 Leverkusen 1, Bayerwerk (DE)**

(72) Erfinder: **Schellhammer, Carl-Wolfgang, Dr.**
**Katharinental 26**
**D - 5060 Bergisch-Gladbach 2 (DE)**
**Wehling, Bernard, Dr.**
**Andreas-Gryphius-Strasse 26**
**D - 5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 461 B1

Pyrazolin-Weißtöner, Verfahren zu ihrer Herstellung sowie ihre Verwendung zum Behandeln von Polyacrylnitril und Wolle

Gegenstand der Erfindung sind Weißtöner der Formel

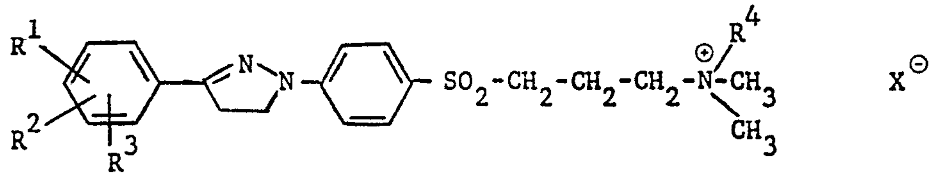

(I)

worin
$R^1$, $R^2$ und $R^3$ Wasserstoff, Chlor oder Methyl,
$R^4$ gegebenenfalls durch Phenyl, Hydroxy, $C_1$—$C_4$-Alkoxy substituiertes $C_1$—$C_4$-Alkyl und
$X^\ominus$ ein farbloses Anion bedeuten.

Diese Verbindungen können durch Umsetzung von Ketonen der Formel

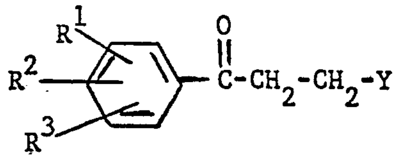

(II)

mit dem Hydrazin der Formel

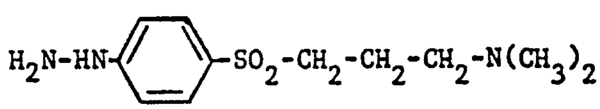

(III)

worin
$R^1$, $R^2$, $R^3$ die oben genannte Bedeutung haben und
Y für Halogen, Di—$C_1$—$C_4$-Alkylamino, Morpholino oder Piperidino steht,

unter anschließender Umsetzung mit einem Alkylierungsmittel hergestellt werden.

Bei der Kondensation werden vorzugsweise mit Wasser mischbare Lösungsmittel wie Alkohole, Äther und Säuren, insbesondere niedere aliphatische Alkohole, Glykole und deren partielle Ester und Äther sowie Essigsäure eingesetzt.

Geeignete Anionen $X^-$ sind beispielsweise Halogenidonen wie Chlorid, Bromid oder Jodid und Sulfonationen wie Methanesulfonat, Äthansulfonat, Benzolsulfonat und Toluolsulfonat.

Dementsprechend verwendet man als Alkylierungsmittel z.B. Methyljodid, Dimethylsulfat, Diäthylsulfat und p-Toluolsulfonsäuremethylester.

Das Hydrazin (III) erhält man durch alkalisch katalysierte Alkylierung von 4-Chlorthiophenol mit 1-Chlor-3-dimethylaminopropan, Oxidation des Thioäthers mit Wasserstoffperoxid in essigsaurer Lösung und Umsetzung des erhaltenen (4-Chlorphenyl)-3-dimethylamino-propyl)-sulfons (IV) mit Hydrazinhydrat unter Rückfluß.

Die Verbindung (IV) kann auch durch Alkylierung des Natriumsalzes der 4-Chlorphenylsulfinsäure mit 1-Chlor-3-dimethylaminopropan dargestellt werden.

Die Weißtöner der Formel (I) eignen sich in hervorragender Weise zum Behandeln von Polyacrylnitril und Wolle. Man erhält Aufhellungen mit guten Echtheiten und hohem Weißgrad.

Gegenüber den nächstvergleichbaren Verbindungen der FR—PS 1296 406 zeigen die erfindungsgemäßen Weißtöner den überraschenden Vorteil der höheren Weißkraft bzw. der verbesserten Lagerstabilität von Flüssigformierungen.

Beispiel 1

10 g der Verbindung der Formel

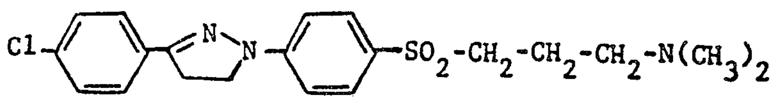

(V)

werden in 300 ml Chlorbenzol bei 50°C gelöst. Man tropft innerhalb von 20 Minuten eine Lösung von 3,4 g Dimethylsulfat in 30 ml Chlorbenzol und rührt 6 Stunden bei 50°C. Man isoliert 12,5 g einer Verbindung der Formel

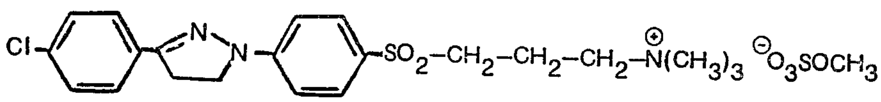

mit dem Schmelzpunkt 226—229°C.

Die freie Base (V) wird auf folgendem Wege dargestellt:

30 g 4-Chlorthiophenol werden mit 24,4 g 1-Chlor-3-dimethylaminopropan und 8,4 g Natriumhydroxid in 800 ml Wasser und 200 ml Äthanol 8 Stunden am Rückfluß gekocht. Das Produkt wird mit Methylenchlorid extrahiert, die Methylenchloridlösung getrocknet, das Lösungsmittel abgezogen und das Produkt destilliert (Siedepunkt: 110—112°C bei 0,1 mm). 40 g 4-Chlorphenyl-3-dimethylaminopropylthioäther werden mit 46 g Wasserstoffperoxid (35%ig) in 300 ml Essigsäure 2 Stunden auf 50°C, dann 2 Stunden auf 100°C und schließlich 5 Stunden auf Rückflußtemperatur erhitzt. Nach Destillation erhält man 35 g 4-Chlorphenyl-3-dimethylaminopropylsulfon, Kp. 170—5°C (0,2 mm).

26 g des 4-Chlorphenyl-3-dimethylaminopropylsulfons werden mit 78 g Hydrazinhydrat 15 Stunden am Rückfluß erhitzt. Nach dem Abkühlen fallen 23 g 4-(3-Dimethylaminopropylsulfonyl)-phenylhydrazin aus, das aus Toluol umkristallisiert bei 113—5°C schmilzt.

10 g 4-(3-Dimethylaminopropylsulfonyl)-phenylhydrazin, 19,8 g einer 40%igen Lösung von 3-Chlor-1-(4-chlorphenyl)-1-propanon in Chlorbenzol und 4,6 g konz. Salzsäure werden in 60 ml Methanol 9 Stunden unter Rückfluß erhitzt. Man distilliert das Lösungsmittel im Vakuum ab, löst den Rückstand in 100 ml Wasser, stellt mit Natronlauge alkalisch, extrahiert mit Methylenchlorid und dampft die organische Phase im Vakuum ein. Nach Umkristallisation des Rückstandes aus Methanol erhält man 11 g des Pyrazolins (V) vom Schmelzpunkt 140—142°C.

Analog lassen sich folgende Quaternierungsprodukte erhalten:

1 - [4 - (3 - Äthyldimethylammonium) - proylsulfonylphenyl] - 3 - (4 - chlorphenyl) - pyrazolin-äthylsulfat vom Fp. 190—193°C.

1 - [4 - (3 - Trimethylammonium) - propylsulfonylphenyl] - 3 - (4 - chlorphenyl) - pyrazolinp - toluolsulfonat vom Fp. 193°C.

1 - [4 - (Benzyldimethylammonium) - propylsulfonylphenyl] - 3 - (4 - chlorphenyl) - pyrazolinchlorid vom Fp. 226°C.

Beispiel 2

Analog Beispiel 1 erhält man durch Kondensation vom 4-(3-Dimethylaminopropylsulfonyl)-phenylhydrazin mit 3-Chlor-1-(3,4-dichlorphenyl)-1-propan das 1-[p-(3-Dimethylamino)-propylsulfonylphenyl]-3-(3,4-dichlorphenyl)-pyrazolin vom Fp. 165—6°C.

Durch Quaternierung wurden folgende Verbindungen gewonnen:

1 - [4 - (3 - Trimethylammonium) - propylsulfonylphenyl] - 3(3,4 - dichlorphenyl) - pyrazolinmethosulfat vom Fp. 218—219°C.

1 - [4 - (3 - Äthyldimethylammonium) - propylsulfonylphenyl] - 3 - (3,4 - dichlorphenyl)-pyrazolin - äthylsulfat vom Fp. 194°C.

1 - [4 - (3 - Trimethylammonium) - propylsulfonylphenyl) - 3 - (3,4 - dichlorphenyl) - pyrazolinp - toluol - sulfonat vom Fp. 193°C.

Beispiel 3

1 - [4 - (3 - Dimethylamino) - propylsulfonylphenyl] - 3 - (4,5 - dichlor - 2 - methylphenyl)-pyrazolin vom Fp. 144—145°C gewinnt man, wie in Beispiel 1 beschrieben, durch Kondensation des Hydrazins (III) mit 3 - Chlor - 1 - (4,5 - dichlor - 2 - methylphenyl) - 1 - propanon. Die Quaternierung führte zu folgenden Verbindungen:

1 - [4 - (3 - Trimethylammonium) - propylsulfonylphenyl] - 3 - (4,5 - dichlor - 2 - methylphenyl)-pyrazolin - methosulfat vom Fp. 234°C.

1 - [4 - (3 - Äthyldimethylammonium) - propylsulfonylphenyl] - 3 - (4,5 - dichlor - 2 - methylphenyl) - pyrazolin - äthylsulfat vom Fp. 188—190°C.

1 - [4 - (3 - Trimethylammonium) - propylsulfonylphenyl] - 3 - (4,5 - dichlor - 2 - methylphenyl)-pyrazolin - p - toluol - sulfonat vom Fp. 187—188°C.

Beispiel 4

Polyacrylnitril-Textilgewebe wird im Flottenverhältnis 1:40 30 Minuten kochend mit einer Färbeflotte behandelt, die 0,3% des nach Beispiel 1 erhaltenen Weißtöners und 3% 30%ige Essigsäure (beides bezogen auf das Textilmaterial) enthält. Nach dem Spülen und Trocknen erhält man ein sehr gut und brillant aufgehelltes Polyacrylnitrilgewebe.

Entsprechend läßt sich nach üblichen Färbeverfahren (Flottenverhältnis 1:40, 60 Minuten bei 55°C und Zusatz von 3 g/l eines handelsüblichen Woll-Bleichmittels) Wolle sehr gut aufhellen. Die übrigen in den Beispielen 1 bis 3 beschriebenen quaternierten Verbindungen bewirken nach den oben aufgeführten Verfahren ebenfalls gute Aufhellungseffekte auf Polyacrylnitrilgewebe und Wolle.

**Patentansprüche**

1. Pyrazolinweißtöner der Formel

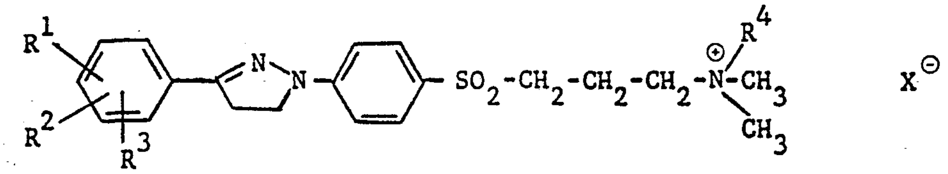

(I)

worin
$R^1$, $R^2$ und $R^3$ Wasserstoff, Chlor oder Methyl,
$R^4$ gegebenenfalls durch Phenyl, Hydroxy oder $C_1$—$C_4$-Alkoxy substituiertes $C_1$—$C_4$-Alkyl und
$X^-$ ein farbloses Anion bedeuten.

2. Verfahren zur Herstellung von Pyrazolinweißtönern gemäß Anspruch 1, dadurch gekennzeichnet, daß man Ketone der Formel

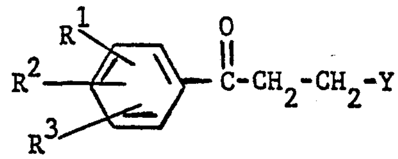

mit dem Hydrazin der Formel

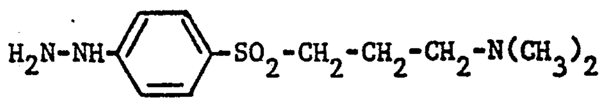

kondensiert, wobei
$R^1$, $R^2$ und $R^3$ die in Anspruch 1 genannte Bedeutung haben und
Y für Halogen, Di—$C_1$—$C_4$-Alkylamino, Morpholino oder Piperidino steht
und anschließend mit einem Alkylierungsmittel umsetzt.

3. Verfahren zum Aufhellen von Polyacrylnitril oder Wolle, dadurch gekennzeichnet, daß man Weißtöner gemäß Anspruch 1 verwendet.

**Claims**

1. Pyrazoline whiteners of the formula

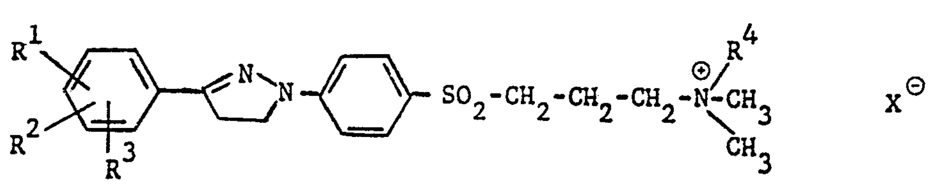

wherein

$R^1$, $R^2$ and $R^3$ denote hydrogen, chlorine or methyl, $R^4$ denotes $C_1$—$C_4$-alkyl which is optionally substituted by phenyl, hydroxyl or $C_1$—$C_4$-alkoxy and $X^-$ denotes a colourless anion.

2. Process for the preparation of pyrazoline whiteners according to Claim 1, characterised in that ketones of the formula

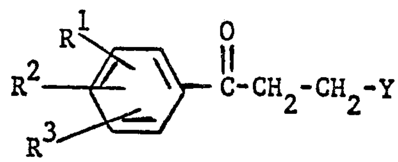

are subjected to a condensation reaction with the hydrazine of the formula

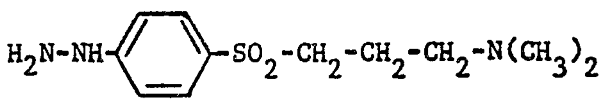

wherein $R^1$, $R^2$ and $R^3$ have the meaning given in Claim 1 and Y represents halogen, di-$C_1$—$C_4$-alkylamino, morpholino or piperidino, and the product is then reacted with an alkylating agent.

3. Process for the brightening of polyacrylonitrile or wool, characterised in that whiteners according to Claim 1 are used.

**Revendications**

1. Azurants optiques pyrazoliniques, caractérisés ce qu'ils répondent à la formule générale:

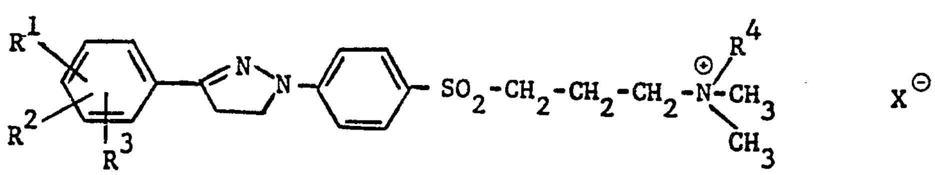
(I)

dans laquelle $R^1$, $R^2$ et $R^3$ représentent chacun un atome d'hydrogène ou de chlore ou un groupe méthyle, $R^4$ est un groupe alkyle en $C_1$—$C_4$ facultativement substitué par un groupe phényle ou hydroxy ou alkoxy en $C_1$—$C_4$ et $X^-$ est un anion incolore.

2. Procédé pour la préparation des azurants selon la revendication 1, caractérisé en ce que l'on condense des cétones de formule générale:

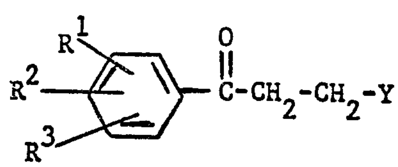

dans laquelle $R^1$, $R^2$ et $R^3$ sont tels que définis ci-dessus et Y est un atome d'halogène ou un groupe di-(alkyl en $C_1$—$C_4$)amino, morpholino et pipéridino avec l'hydrazine de formule

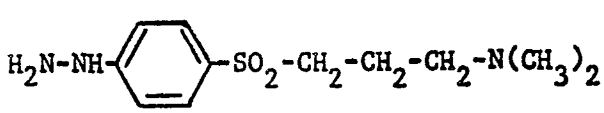

et on fait ensuite réagir avec un agent alkylant.

3. Procédé pour l'azurage optique de polyacrylonitrile ou de laine caractérisé en ce qu'on utilise des azurants optiques selon la revendication 1.